**Europäisches Patentamt**

**European Patent Office**

**Office européen des brevets**

(19)

(11) Veröffentlichungsnummer: **0 470 344 B1**

## (12) EUROPÄISCHE PATENTSCHRIFT

(45) Veröffentlichungstag der Patentschrift: **12.04.95**

(21) Anmeldenummer: **91109648.5**

(22) Anmeldetag: **12.06.91**

(51) Int. Cl.6: **C07C 29/141**, C07C 29/17, C07C 31/125, C07C 31/12

(54) **Verfahren zur Herstellung von gesättigten Alkoholen aus Aldehyden.**

(30) Priorität: **09.08.90 DE 4025245**

(43) Veröffentlichungstag der Anmeldung:
**12.02.92 Patentblatt 92/07**

(45) Bekanntmachung des Hinweises auf die Patenterteilung:
**12.04.95 Patentblatt 95/15**

(84) Benannte Vertragsstaaten:
**DE FR IT SE**

(56) Entgegenhaltungen:
**EP-A- 0 420 035**
**FR-A- 1 293 317**
**US-A- 3 886 219**

(73) Patentinhaber: **HÜLS AKTIENGESELLSCHAFT**

**D-45764 Marl (DE)**

(72) Erfinder: **Ludwig, Gerhard, Dr.**
**Holtweg 23**
**W-4358 Haltern 6 (DE)**
Erfinder: **Fischer, Lothar, Dr.**
**Leverkusener Strasse 15**
**W-4370 Marl (DE)**
Erfinder: **Hess, Dieter, Dr.**
**Sickingmühler Strasse 36a**
**W-4370 Marl (DE)**

Anmerkung: Innerhalb von neun Monaten nach der Bekanntmachung des Hinweises auf die Erteilung des europäischen Patents kann jedermann beim Europäischen Patentamt gegen das erteilte europäische Patent Einspruch einlegen. Der Einspruch ist schriftlich einzureichen und zu begründen. Er gilt erst als eingelegt, wenn die Einspruchsgebühr entrichtet worden ist (Art. 99(1) Europäisches Patentübereinkommen).

**Beschreibung**

Die Erfindung betrifft ein Verfahren zur Herstellung von gesättigten Alkoholen durch Hydrierung von Aldehyden in der Gasphase in mehreren Stufen, wobei in der ersten Stufe ein alkalisch eingestellter Kupferkatalysator und in der zweiten Stufe ein Nickel enthaltender Katalysator eingesetzt wird.

Es ist bekannt, daß man Alkohole durch katalytische Hydrierung der entsprechenden gesättigten und ungesättigten Aldehyde herstellen kann.

So wird in DE-A-21 50 975 die Herstellung und Anwendung eines hochaktiven Nickelkatalysators auf einem $SiO_2$-Träger beschrieben, der weniger als 0,2 % Natrium enthält und auch zur Hydrierung von Aldehyden geeignet ist. Dabei wird beispielsweise 2-Ethylhexanal bei einem geringen Wasserstoff-Aldehyd-Molverhältnis von nur 2,5 : 1 bei 36 bar in der Flüssigphase hydriert. Der Umsatz liegt bei 96 %.

Bei Hydrierungen in der Flüssigphase sind Anlagendrücke von 20 bis 300 bar üblich, um eine genügend vollständige Hydrierung zu erzielen. Da die Reaktion stark exotherm ist, ist in technischen Reaktoren die Rückführung eines beträchtlichen Teils des hydrierten Produktes oder die Verdünnung mit einem Lösemittel zur kapazitiven Wärmeabfuhr erforderlich. Dies läßt nur verhältnismäßig niedrige Raumbelastungen der Reaktoren mit Aldehyden zu.

Diese Schwierigkeiten werden durch eine Hydrierung in der Gasphase vermieden. So wird in DE-C-1 152 393 eine Gasphasenhydrierung in zwei Stufen durchgeführt. Dabei wird in der ersten Stufe ein Kupferkatalysator und in der zweiten Stufe ein Kupfer-Nickel-Katalysator verwendet. Bei der Gasphasenhydrierung von DE-C-1 161 250 wird in der ersten Stufe ein Kupferkatalysator und in der zweiten Stufe ein Nickelkatalysator eingesetzt. In beiden Patenten werden als Träger der Katalysatoren Kieselgur oder mit Natriumphosphat modifizierte Kieselgur verwendet. Die Oberfläche dieser Träger ist neutral oder schwach alkalisch.

DE-B-1 227 882 beschreibt eine zweistufige Gasphasenhydrierung an einem Kupfer- und einem Palladiumkatalysator, dabei kann auch ein Kupfer-Nickel-Katalysator zwischengeschaltet werden. Man verwendet hier nur neutrale oder schwach alkalische Träger. Die Kupfer- und Nickelgehalte der Trägerkatalysatoren sind sehr hoch. Außerdem weisen die Produkte noch beträchtliche Restaldehydgehalte auf.

In DE-C-1 276 620 wird eine einstufige Hydrierung in der Gasphase an einem Kupfer-Nickel-Trägerkatalysator beschrieben. Von einer zweistufigen Hydrierung an einem Kupfer- und danach an einem Kupfer-Nickel-Katalysator wird allerdings gemäß Vergleichsbeispiel abgeraten.

Nach DE-C-1 276 618 kann die Gasphasenhydrierung zweistufig durchgeführt werden. Dabei wird hier in der ersten Stufe ein Kupfer-Nickel-Katalysator und in der zweiten Stufe ein Nickel- oder Palladium-Katalysator verwendet.

Eine weitere Verbesserung der Gasphasenhydrierung wird in DE-C-1 643 856 beschrieben. Hier wird die Hydrierung an Kupfer und/oder Nickel enthaltenden Kieselsäuregel-Trägerkatalysatoren durchgeführt. Dabei wird der pH-Wert der Oberfläche des Kieselsäuregels auf 6 bis 10 eingestellt. Der "Oberflächen-pH-Wert" wird u.a. nach der Methode von O. Johnson, J. Phys. Chem. 59, 827 (1955), bestimmt. Für schwer hydrierbare Aldehyde und bei hoher Katalysatorbelastung können auch übliche Nickel- und/oder Palladium-Trägerkatalysatoren nachgeschaltet werden. Obwohl zum Oberflächen-pH-Wert der nachgeschalteten Katalysatoren keine Aussagen gemacht werden, legt das Patent auch hier einen pH-Wert von 6 bis 10 nahe, zumal in Vergleichsbeispielen eine Abgrenzung gegen Katalysatoren vorgenommen wird, deren Oberflächen-pH-Wert unter 6 beziehungsweise über 10 liegt.

Wir haben gefunden, daß hier bei hoher Katalysatorbelastung die Bildung von Kohlenwasserstoffen stark zunimmt und die Selektivität deutlich abfällt.

In DE-A-37 37 277 wird die Hydrierung, die vorzugsweise in zwei Druckstufen abläuft, an einem Kupfer/Zinkoxid-Katalysator, dem ein Alkalimetall und/oder ein Übergangsmetall zudotiert ist, durchgeführt. Dabei wird der Katalysator vorzugsweise mit Kalium und Nickel imprägniert. Nach dieser Patentanmeldung wird die Hydrierung durch den Einsatz eines alkalisch eingestellten Katalysators verbessert.

Es bestand nun die Aufgabe, bei der Gasphasenhydrierung von gesättigten und ungesättigten Aldehyden zu gesättigten Alkoholen Ausbeute und Selektivität weiter zu verbessern. Insbesondere sollten auch bei hohen Katalysatorbelastungen Rohalkohole hergestellt werden, die den hohen Anforderungen an Bromzahl und Schwefelsäurefarbzahl genügen.

Die Aufgabe wird erfindungsgemäß dadurch gelöst, daß man eine mehrstufige Hydrierung gemäß Anspruch 1 durchführt.

Dabei werden in der ersten Stufe mindestens 85 % der Hydrierreaktionen an einem körnigen, alkalisch eingestellten Kupfer-Katalysator durchgeführt. In der zweiten Stufe verwendet man einen körnigen, Nickel enthaltenden Trägerkatalysator, wobei der Träger eine saure Oberfläche aufweist. Die Oberfläche wird dadurch charakterisiert, daß sie mindestens 0,05 mmol/g saure Zentren enthält, die eine Säurestärke $H_o$ von

2,8 bis 4,8 aufweisen. Der Katalysator der zweiten Stufe liegt in Korngrößen von 1 bis 10 mm Durchmesser vor, wobei diese Grenzen besagen, daß über 90 Gew.-% des Katalysators in diesem Bereich liegen müssen. Vorzugsweise weist der Katalysator der zweiten Stufe Korngrößen von 2 bis 7 mm auf.

Körnige Katalysatoren im Sinne der vorliegenden Erfindung sind beispielsweise Extrudate, Tabletten, Ringe, Kugeln oder unregelmäßiges Granulat.

Der Kupferkatalysator der ersten Reaktionsstufe kann nach üblichen Methoden als Fäll- oder Imprägnierkatalysator hergestellt werden. Er enthält zweckmäßigerweise übliche Promotoren wie z. B. Chromoxid, Manganoxid, Molybdänoxid, Phosphat, einzeln oder im Gemisch, sowie mindestens 0,5 % Alkali. Der Kupfergehalt kann zwischen ca. 3 und 80 % liegen. Vorzugsweise wählt man einen Imprägnierkatalysator mit 5 bis 15 % Kupfer. Als Trägermaterialien kommen Oxide des Siliciums, Aluminiums, Titans oder Zinks oder Gemische derselben in Frage. Wichtig ist, daß die Oberfläche dieses Katalysators keine freien sauren Zentren hat.

An diesem ersten Katalysator läuft die Reaktion vorzugsweise zu 90 - 98 % ab. Dies bezieht sich auf die Summe aller hydrierbaren Doppelbindungen.

Der Umsatz der ersten Stufe wird durch die Parameter Eingangstemperatur, Konzentration des Aldehyds, $H_2$-Partialdruck, Lineargeschwindigkeit der Reaktionskomponenten und die Art und Wirksamkeit der Wärmeableitung bestimmt. Das komplexe Reaktionsgeschehen macht eine Vorhersage der notwendigen Bedingungen für die Erzielung des gewünschten Umsatzes unmöglich. Der Umsatz wird deshalb empirisch bestimmt. So kann man beispielsweise die vorgesehene Menge Kupferkatalysator allein in einen Testreaktor einfüllen und die variierbaren Reaktionsparameter solange verändern, bis die gewünschte Umsatzgröße erreicht ist. Die so gefundenen Parameter überträgt man auf den technischen Reaktor.

Eine andere Methode beruht darin, daß man an der Grenze zwischen dem Kupfer- und dem nachfolgenden Katalysator in der Wandung des Reaktors einen Probeentnahmestutzen anbringt und an Hand der an dieser Stelle ermittelten Produktzusammensetzung die variierbaren Parameter so justiert, daß die erfindungsgemäße Umsatzgröße in der ersten Stufe erreicht wird.

Nach Eichung durch eine der vorgenannten Methoden ist die Messung des Temperaturverlaufs im Reaktor im allgemeinen ein genügend genaues Überwachungs- und Steuerungsinstrument.

Der Nickel enthaltende Katalysator der zweiten Reaktionsstufe ist vorzugsweise ein reiner Nickelkatalysator oder ein Kupfer-Nickel-Katalysator, bei dem das Massenverhältnis von Kupfer : Nickel im Bereich von 5 : 1 bis 1 : 5 liegt.

Der Nickel enthaltende Katalysator kann beispielsweise durch Fällung oder durch Tablettierung von pulverförmigen Katalysatormaterial hergestellt werden. Sehr gute Katalysatoren erhält man, wenn man das körnige Trägermaterial mit einer Metallsalzlösung behandelt und dann trocknet. Dabei kann die aktive Masse überwiegend in der Randzone aufgebracht werden. Derartige randzonenimprägnierte Katalysatoren werden vorzugsweise verwendet.

Der Gesamtmetallgehalt des Katalysators der zweiten Stufe ist keine kritische Größe. Er kann etwa 3 bis 30 % betragen. Im Falle eines randzonenimprägnierten Katalysators liegt der Metallgehalt meist bei 10 bis 20 %.

Der Anteil des Katalysators der zweiten Stufe am gesamten Katalysatorvolumen beträgt vorzugsweise 20 bis 70 %, wobei Gehalte von 25 bis 45 % besonders bevorzugt werden.

Der Nickel enthaltende Katalysator enthält im allgemeinen übliche Promotoren, wie z. B. Chromoxid, Manganoxid, Molybdänoxid oder Phosphat, in einer Gesamtmenge von bis zu 3 %, bezogen auf den Trägerkatalysator. Dabei werden Gehalte von 0,3 bis 2 % bevorzugt.

Das Trägermaterial besteht vorzugsweise aus Aluminiumoxid oder Siliciumdioxid, wobei körniges Kieselgel mit relativ großer spezifischer innerer Oberfläche besonders bevorzugt wird.

Das Trägermaterial besitzt vorzugsweise eine spezifische innere Oberfläche nach Brunauer-Emmett-Teller (BET-Methode) von über 150 $m^2$/g, wobei spezifische innere Oberflächen von über 200 $m^2$/g ganz besonders bevorzugt werden.

Für die zweite Reaktionsstufe wird vorzugsweise ein Trägermaterial verwendet, das 0,1 bis 0,4 mmol/g saure Zentren aufweist. Diese Bedingung wird im allgemeinen durch Aluminiumoxid und Siliciumdioxid, das gut mit Säuren und Wasser gewaschen ist und einen Natriumgehalt von bis zu 0,3 % aufweist, erfüllt.

Die Definition eines Oberflächen-pH-Wertes nach O. Johnson ist problematisch, da bei Oberflächen kaum von Wasserstoffionenkonzentrationen gesprochen werden kann. Wir verwenden deshalb die Definitionen und Meßmethoden von H.A. Benesi, J. Phys. Chem. 61, 970 (1957), um die "Zahl und Stärke" der sauren Zentren der Trägermaterialien zu charakterisieren.

Den Säurestärken $H_o$ werden dabei Farbindikatoren mit bestimmten $pK_a$-Werten zugeordnet. Methylrot mit einem $pK_a$-Wert von 4,8 markiert dabei die eine Grenze. Bei noch höheren $pK_a$-Werten ist die Säurestärke $H_o$ nicht ausreichend. Der Farbindikator 4-Aminoazobenzol mit einem $pK_a$-Wert von 2,8 kennzeichnet dabei die andere Grenze.

Bei einer so definierten Säurestärke $H_o$ herrscht bei 0,05 mmol/g sauren Zentren eine Oberflächenacidität, die einem sogenannten Oberflächen-pH-Wert (nach DE-C-1 643 856) von ca. 4 entspricht.

Das Hydrierverfahren wird in mehreren Stufen durchgeführt, vorzugsweise in zwei oder drei Stufen, wobei man in der dritten Stufe einen körnigen Palladiumkatalysator verwenden kann. Vorzugsweise verwendet man hier Trägerkatalysatoren, auf denen 0,05 bis 5 % Palladium abgeschieden wurden. Randzonenimprägnierte Katalysatoren mit einem Palladiumgehalt von 0,3 bis 0,8 % werden dabei ganz besonders bevorzugt.

Der Träger des Palladiumkatalysators besitzt vorzugsweise eine saure Oberfläche mit über 0,05 mmol/g sauren Zentren. Auch hier werden Träger mit 0,1 bis 0,4 mmol/g sauren Zentren besonders bevorzugt.

Bezogen auf das Katalysatorgesamtvolumen beträgt das Volumen des Palladiumkatalysators meist 5 bis 20 %, wobei ein Anteil von 8 bis 15 % bevorzugt wird.

Die einzelnen Stufen der Hydrierung können in mehreren, nacheinander geschalteten Reaktoren ausgeführt werden. Vorzugsweise werden die Katalysatoren jedoch in einem einzigen Reaktor mit oder ohne Trennböden, mit oder ohne Zwischenkühlungseinrichtungen derart übereinandergeschichtet, daß der Kupferkatalysator als erster vom Reaktionsgemisch durchströmt wird.

Das Verfahren kann in einem einzigen adiabatisch arbeitenden Schachtreaktor (gegebenenfalls mit Zwischenkühlung) oder in einem z. B. druckwassergekühlten Vielrohrreaktor durchgeführt werden.

Als Aldehyde können aliphatische gesättigte und ungesättigte Aldehyde mit 2 bis 12 C-Atomen eingesetzt werden. Dabei können relativ reine Aldehyde oder auch durch vorauflaufende Prozesse verunreinigte Rohaldehyde eingesetzt werden. Gelegentlich ist auch die Verdünnung der Aldehyde, z. B. durch Alkohole mit gleicher oder niedriger C-Zahl, von Vorteil.

Beispiele für geeignete Aldehyde sind Acetaldehyd, Propionaldehyd, Acrolein, Butyraldehyd, Crotonaldehyd, Hexanal, 2-Ethylhexanal, 2-Ethylhexenal, Nonanal oder Decanal.

Zur Hydrierung wird der Aldehyd zuerst verdampft. Dabei wird Wasserstoff im Überschuß zugemischt. Das molare Verhältnis von Wasserstoff zu Aldehyd beträgt vorzugsweise 100 : 1 bis 3 : 1. Ein Verhältnis von 30 : 1 bis 5 : 1 wird besonders bevorzugt.

Bei der Einstellung der Gasgemische ist darauf zu achten, daß der Taupunkt der Gemische niedriger liegt als die Reaktoreingangstemperatur, für die vorzugsweise eine Temperatur im Bereich von 125 bis 160 °C gewählt wird.

Der Taupunkt hängt auch vom Anlagendruck ab. Die Hydrierung kann bei Normaldruck durchgeführt werden. Man kann jedoch auch einen geringen Unterdruck bis 500 mbar oder einen Überdruck bis 20 bar anlegen.

Das vorliegende Verfahren hat folgende Vorteile:

- Die Hydrierung kann in einem einzigen Reaktor durchgeführt werden. Spezielle bauliche Veränderungen an bekannten Hydrierreaktoren sind nicht erforderlich.
- Bei gleichbleibender Qualität des Hydrieraustrags kann die Belastung des Katalysators und der Partialdruck des Aldehyds deutlich erhöht werden.
- Bei der gleichen Katalysatorbelastung wie bei bekannten Verfahren wird die Selektivität erhöht. Die Bildung von Kohlenwasserstoffen, Ethern und Estern wird weiter zurückgedrängt.

Nachfolgend wird angegeben, wie die Erfindung praktisch ausgeführt werden kann.


Bestimmung der Zahl der sauren Zentren an Trägermaterialien:


15 g Trägermaterial wird pulverisiert und 12 Stunden bei 250 °C im Vakuum getrocknet. In 10 vorher gewogene, verschraubbare Flaschen werden jeweils ca. 1 g Probe gefüllt. Die verschlossenen Flaschen werden zurückgewogen. Danach werden die Proben mit jeweils 10 ml wasserfreiem Cyclohexan überschichtet und mit 0,05 bis 0,5 mmol n-Butylamin in 0,05 mmol-Abstufungen durch Zugabe entsprechender Mengen einer 0,05 molaren Lösung von n-Butylamin in wasserfreiem Cyclohexan versetzt. Die dicht verschlossenen Gläser werden anschließend 24 Stunden geschüttelt.

Je 2 ml Suspension werden in Schnappdeckelgläser gefüllt und mit 2 Tropfen Indikatorlösung versetzt. Die Gläser werden in der Reihe steigenden n-Butylamingehalts angeordnet. Nach einer halben Stunde wird an Hand des Glases, das als erstes Farbumschlag zeigt, die zur Neutralisation der Säurezentren erforderliche n-Butylaminmenge als Äquivalenz der Zahl der Säurezentren bestimmt.

Herstellung der Katalysatoren:

Für die Katalysatoren A, B und C werden als Träger Kieselgelperlen mit 3 bis 5 mm Durchmesser mit einem Porenvolumen von 0,7 $cm^3$/g und einer spezifischen inneren Oberfläche von 300 $cm^2$/g verwendet. Nach der Benesi-Methode wird mit Methylrot als Indikator die Zahl der sauren Zentren mit 0,15 mmol/g gemessen. Die Säurestärke $H_o$ beträgt $\leq$ 4,8. 4-Aminoazobenzol ($pK_a$ = 2,8) zeigt keinen Farbumschlag.

Der alkalische Katalysator A wird durch Imprägnieren des Trägers mit ammoniakalischer chromathaltiger Kupfercarbonatlösung hergestellt. Der Imprägnierlösung werden außerdem pro kg Träger 0,30 mol Natronlauge hinzugefügt, so daß die sauren Zentren zu 100 % überkompensiert werden. Nach der Imprägnierung wird der Katalysator getrocknet.

Der DE 1 643 846 entsprechende Katalysator B wird in gleicher Weise mit einer Kupfer- und Nickelnitrat sowie Chromat enthaltenden Imprägnierlösung hergestellt. Er wird durch einen Zusatz von 0,15 mol Natronlauge pro kg Träger neutral eingestellt.

Zur Herstellung des erfindungsgemäßen Katalysators C werden 72 kg Kieselgelperlen in einer rotierenden Trommel bei ca. 100 °C mit 104 kg einer wäßrigen Lösung, die 9,2 % $Cu^{2+}$, 3,95 % $Ni^{2+}$ und 0,923 % Cr (VI) als Chromat und 26,4 % $NO_3^-$ enthält, imprägniert. Dabei werden die aktiven Komponenten in einer Randzone von 0,2 bis 1 mm abgeschieden. Die so beladenen Perlen werden im Luftstrom zuerst getrocknet und danach bei Temperaturen bis 320 °C calciniert.

Der Palladiumkatalysator D hat als Träger strangförmiges Aluminiumoxid mit einem Durchmesser von 1,6 mm, einem Porenvolumen von 0,7 $cm^3$/g und einer spezifischen inneren Oberfläche von 230 $m^2$/g. Das Aluminiumoxid weist nach der Benesi-Methode 0,25 mmol/g saure Zentren sowie eine Säurestärke $H_o$ von $\leq$ 4,8 auf.

Das Aluminiumoxid wird unter Rotieren in einer Trommel bei ca. 100 °C mit Palladiumnitrat imprägniert, wobei man pro kg Aluminiumoxid 1 l einer 0,7 %igen Palladiumnitratlösung verwendet. Das Palladium wird dabei in einer 0,01 bis 0,1 mm dicken Randzone auf dem Träger abgeschieden. Die beladenen Stränge werden im Luftstrom bei 110 °C getrocknet und danach bei Temperaturen bis 450 °C calciniert.

Tabelle 1

| | | | | | | Katalysatoren |
|---|---|---|---|---|---|---|
| | Träger | Cu % | Ni % | $Cr_2O_3$ % | Pd % | Zahl der sauren Zentren im Träger mmol/g |
| Katalysator A | $SiO_2$ | 9 | - | 1,4 | - | 0 (alkalisch) |
| Katalysator B | $SiO_2$ | 10,4 | 4,6 | 1,4 | - | 0 (neutral) |
| Katalysator C | $SiO_2$ | 10,5 | 4,5 | 1,5 | - | 0,15 (sauer) |
| Katalysator D | $Al_2O_3$ | - | - | - | 0,66 | 0,25 (sauer) |

Versuchsapparatur:

Der Versuchsreaktor besteht aus einem mit Druckwasserzwangsumlauf temperierten ummantelten Rohr aus Edelstahl. Der Innendurchmesser des Rohres beträgt 67 mm, die Länge ca. 6,0 m. Zur Temperaturmessung sind im Abstand von 50 cm Thermoelemente in einem zentral installierten Innenrohr von 8 mm Durchmesser angebracht, so daß der freie Querschnitt 34,9 $cm^2$ beträgt. Der Reaktor wird bis zu einer Höhe von 5,9 m mit Katalysator befüllt, was einem Gesamtkatalysatorvolumen von 20,4 l entspricht.

Vor dem Reaktor befindet sich ein mantelbeheizter Verdampfer, in dem die Einsatzprodukte mit heißem zirkulierendem Gas verdampft werden. Das Gas wird mit Hilfe eines Kreisgasverdichters umgewälzt.

Das hydrierte Produkt wird nach dem Reaktor durch einen wassergekühlten Kondensator auf weniger als 20 °C abgekühlt und flüssig abgezogen.

Probestutzen erlauben die Abnahme von Analysenmaterial vor und nach dem Reaktor sowie in verschiedenen Höhen der Katalysatorschicht. Die Gasströmung verläuft im Reaktor von oben nach unten.

Die Manteltemperatur des Reaktors kann mit einer Schwankungsbreite von ± 2 °C geregelt werden.

Einsatzmaterialien:

a) 2-Ethylhexenal: Verwendet wird rohes Produkt aus der Aldolkondensation einer Großanlage, das durch Dekantation von der Kondensationslauge abgetrennt war. Der 2-Ethylhexenal-Gehalt beträgt ca. 96 %.

b) n-Butyraldehyd: Bei diesem handelte es sich um destilliertes Handelsprodukt mit einem Gehalt von 99,4 - 99,6 %.

c) iso-Butyraldehyd: Dieser Aldehyd ist ein destilliertes Handelsprodukt mit einem Gehalt von 99,8 %.

Analytik:

Bromzahl: ASTM D 1159-77

Schwefelsäurefarbzahl: International Standard (ISO) 1843/8

In den Versuchen A und B sowie 1 und 2 wurden die Schwefelsäurefarbzahlen am rohen Hydrieraustrag ermittelt.

Die gaschromatographische Untersuchung der Produkte wurde an einer 12-m-Kapillare vom Typ OV 101 (Siliconöl) mit einem Temperaturgradienten von 50 bis 250 °C und einer Aufheizgeschwindigkeit von 8 °C/min vorgenommen. Bei der Untersuchung des 2-Ethylhexanols wurde eine zweite Kapillarsäule von 25 m Länge vom Typ CW (Carbowachs) mit gleicher Aufheizcharakteristik zusätzlich verwendet.

Neben den Alkoholgehalten wurden gaschromatographisch die Gehalte an Kohlenwasserstoffen mit um 1 niedrigerer Kohlenstoffzahl, an gesättigten Aldehyden sowie die Anteile an Ethern und Estern mit doppelter Kohlenstoffzahl bestimmt.

**Versuche:**

Die Versuche wurden in dem oben beschriebenen Reaktor bei einem Wasserstoffdruck von 6,0 bar abs. mit einem Gasvolumenstrom GHSV (gaseous hourly space velocity) von 900 $h^{-1}$ (= 900 Norm-$m^3$ $H_2$ pro $m^3$ Katalysator und Stunde) durch geführt.

Nachfolgend wurden Vergleichsversuche durch Buchstaben und erfindungsgemäße Versuche durch Zahlen gekennzeichnet.

In Versuch A wurde der alkalisch eingestellte Kupferkatalysator A als einziger Katalysator verwendet. In den Versuchen 1 und 2 wurde er als erster Katalysator eingesetzt. In den Versuchen 1 und 2 wurde außerdem darauf geachtet, daß ca. 90 bis 95 % des Umsatzes der vorhandenen C = C - und C = 0 - Bindungen an diesem Katalysator erfolgten.

In Versuch B wird nur der neutral eingestellte Katalysator B verwendet. Dieser Versuch stellt im wesentlichen eine Wiederholung von Beispiel 1 aus DE 1 643 856 dar. Es wurden lediglich ein niedrigerer Druck, eine geringere Lineargeschwindigkeit und z. T. eine höhere Gasbelastung eingestellt.

Versuch A

Hydrierung von 2-Ethylhexenal
Katalysator: 100 Vol. % A

## Tabelle 2:

| | LHSV 1/h | Verhältnis $H_2$:EH | Reaktor-Eingang °C | Reaktor-Mantel °C |
|---|---|---|---|---|
| a | 0,15 | 39 : 1 | 135 | 160 |
| b | 0,20 | 30 : 1 | 135 | 160 |
| c | 0,25 | 24 : 1 | 135 | 165 |
| d | 0,30 | 20 : 1 | 140 | 165 |

| | Brom-zahl mgBr/ 100 g | $H_2SO_4$-Farbz. | n-Heptan % | 2-Ethyl-hexanal % | 2-Ethyl-hexanol % | Dioctyl-ether % | Dioctyl-ester % |
|---|---|---|---|---|---|---|---|
| a | 0,1-0,3 | 300 | - | 0,2 | 95,1 | - | 0,1-0,2 |
| b | 0,2-0,4 | >1000 | - | 0,3 | 95,0 | - | 0,2-0,5 |
| c | 0,3-0,7 | >1000 | 0,02 | 0,3-0,4 | 94,8 | - | 0,3-0,8 |
| d | 0,5-1,0 | >1000 | 0,02 | 0,3-0,4 | 93,7 | 0,02 | 0,7-2,1 |

LHSV = Flüssigkeitsvolumenstrom des vergasten Produkts (liquid hourly space velocity) ($m^3$ Flüssigaldehyd pro $m^3$ Katalysator und Stunde)

EH = 2-Ethylhexenal

Wie man dieser Tabelle entnehmen kann, sind die Schwefelsäurefarbzahlen und bei höherer Belastung zusätzlich die Estergehalte sehr hoch.

Versuch B

Hydrierung von 2-Ethylhexenal
Katalysator: 100 Vol. % B

## Tabelle 3:

|   | LHSV 1/h | Verhältnis $H_2$:EH | Reaktor-Eingang °C | Reaktor-Mantel °C |
|---|---|---|---|---|
| a | 0,15 | 39 : 1 | 135 | 160 |
| b | 0,20 | 30 : 1 | 135 | 160 |
| c | 0,25 | 24 : 1 | 135 | 160 |
| d | 0,30 | 20 : 1 | 140 | 165 |
| e | 0,35 | 17 : 1 | 140 | 165 |

|   | Brom-zahl mgBr/ 100 g | $H_2SO_4$-Farbz. | n-Heptan % | 2-Ethyl-hexanal % | 2-Ethyl-hexanol % | Dioctyl-ether % | Dioctyl-ester % |
|---|---|---|---|---|---|---|---|
| a | 0,05 | 150 | 0,1-0,2 | 0,2 | 95,1 | 0,03 | < 0,1 |
| b | 0,07 | 150-250 | 0,2-0,4 | 0,3 | 95,0 | 0,08 | 0,1 |
| c | 0,1-0,2 | 150-300 | 0,3-0,6 | 0,3-0,4 | 94,8 | 0,07 | 0,1 |
| d | 0,1-0,3 | > 300 | 1,0-2,0 | 0,3-0,5 | 93,5 | 0,1 | 0,1-0,3 |
| e | 0,2-0,5 | > 300 | > 2,5 | 0,7 | < 93 | 0,1-0,3 | 0,1-0,3 |

Wie diese Tabelle zeigt, ist die n-Heptanbildung ab einer LHSV von 0,2 $h^{-1}$ sehr hoch.

Versuch 1

Hydrierung von 2-Ethylhexenal
Katalysatorkombination: 60 Vol. % A + 40 Vol. % C

Tabelle 4:

| | LHSV<br><br>1/h | Verhältnis<br>$H_2$:EH | Reaktor-<br>Eingang<br>°C | Reaktor-<br>Mantel<br>°C |
|---|---|---|---|---|
| a | 0,15 | 39 : 1 | 135 | 160 |
| b | 0,20 | 30 : 1 | 135 | 160 |
| c | 0,25 | 24 : 1 | 135 | 160 |
| d | 0,30 | 20 : 1 | 140 | 165 |
| e | 0,35 | 17 : 1 | 140 | 165 |
| f | 0,40 | 15 : 1 | 140 | 165 |

| | Brom-<br>zahl<br>mgBr/<br>100 g | $H_2SO_4$-<br>Farbz. | n-<br>Heptan<br><br>% | 2-Ethyl-<br>hexanal<br><br>% | 2-Ethyl-<br>hexanol<br><br>% | Dioctyl-<br>ether<br><br>% | Dioctyl-<br>ester<br><br>% |
|---|---|---|---|---|---|---|---|
| a | 0,01 | 50 | 0,02 | 0,2 | ≥ 96 | ≤ 0,03 | < 0,1 |
| b | 0,02 | 50-100 | 0,05 | 0,3 | 95,7 | ≤ 0,03 | < 0,1 |
| c | 0,02-0,04 | 100-200 | 0,05-0,10 | 0,3 | 95,6 | ≤ 0,03 | 0,2 |
| d | 0,03-0,10 | 100-200 | 0,05-0,10 | 0,4 | 95,3 | ≤ 0,03 | 0,2 |
| e | 0,1 | 150-300 | 0,05-0,15 | 0,5 | 95,3 | ≤ 0,03 | 0,2 |
| f | 0,1-0,2 | 200-300 | 0,1-0,2 | 0,5 | 96,0 | ≤ 0,03 | 0,3 |

In Versuch f bei der Belastung von 0,4 $h^{-1}$ werden bei der Verdampfung 1 - 2 % Rückstand aus dem Verdampfer ausgeschleust.

Versuch 2

Hydrierung von 2-Ethylhexenal
Katalysatorkombination: 60 Vol. % A + 30 Vol. % C + 10 Vol. % D

Tabelle 5:

| · | LHSV 1/h | Verhältnis H$_2$:EH | Reaktor-Eingang °C | Reaktor-Mantel °C |
|---|---|---|---|---|
| a | 0,15 | 39 : 1 | 135 | 160 |
| b | 0,20 | 30 : 1 | 135 | 160 |
| c | 0,25 | 24 : 1 | 135 | 160 |
| d | 0,30 | 20 : 1 | 140 | 160 |
| e | 0,35 | 17 : 1 | 140 | 160 |
| f | 0,40 | 15 : 1 | 140 | 160 |

| | Brom-zahl mgBr/ 100 g | H$_2$SO$_4$-Farbz. | n-Heptan % | 2-Ethyl-hexanal % | 2-Ethyl-hexanol % | Dioctyl-ether % | Dioctyl-ester % |
|---|---|---|---|---|---|---|---|
| a | 0,008 | 50 | 0,02 | 0,2 | 96,2 | ≤ 0,03 | < 0,1 |
| b | 0,01 | 50 | 0,05 | 0,2 | 95,9 | ≤ 0,03 | < 0,1 |
| c | 0,01-0,02 | 50-150 | 0,10 | 0,2 | 95,8 | ≤ 0,03 | 0,1 |
| d | 0,02-0,05 | 100-200 | 0,05-0,10 | 0,2 | 95,4 | ≤ 0,03 | 0,2 |
| e | 0,05-0,15 | 100-250 | 0,05-0,20 | 0,3 | 95,4 | ≤ 0,03 | 0,2 |
| f | 0,02-0,10 | 100-200 | 0,1-0,2 | 0,2 | 96,2 | ≤ 0,03 | 0,3 |

In Versuch f bei der Belastung von 0,4 h$^{-1}$ werden bei der Verdampfung 1 - 2 % Rückstand aus dem Verdampfer ausgeschleust.

Die Versuche 1 und 2 zeigen im Vergleich zu Versuch A und B deutlich die Verbesserung hinsichtlich Selektivität, Farb- und Bromzahl.

Der Versuch 2 lief unter wechselnden Bedingungen, meist mit höchster Belastung, insgesamt mehr als 500 Tage.

Versuch 3

Hydrierung von n-Butyraldehyd
Katalysatorkombination: 60 Vol. % A + 30 Vol. % C + 10 Vol. % D

## Tabelle 6:

| | LHSV 1/h | Verhältnis $H_2$:n-BA | Reaktor-Eingang °C | Reaktor-Mantel °C |
|---|---|---|---|---|
| a | 0,30 | 12 : 1 | 130 | 160 |
| b | 0,40 | 9 : 1 | 130 | 160 |
| c | 0,50 | 7 : 1 | 130 | 160 |
| d | 0,60 | 6 : 1 | 130 | 165 |

| | n-Butyr-aldehyd % | n-Butanol % | Dibutyl-ether % | Butyl-butyrat % |
|---|---|---|---|---|
| a | 0,1 | 99,5 | 0,01 | 0,05 |
| b | 0,2 | 99,5 | 0,03 | 0,1 |
| c | 0,2 | 99,4 | 0,04 | 0,2 |
| d | 0,3 | 99,2 | 0,06 | 0,3 |

Versuch 4

Hydrierung von iso-Butyraldehyd
Katalysatorkombination: 60 Vol. % A + 30 Vol. % C + 10 Vol. % D

## Tabelle 7:

| | LHSV<br><br>1/h | Verhältnis<br>$H_2$:iso-BA | Reaktor-<br>Eingang<br>°C | Reaktor-<br>Mantel<br>°C |
|---|---|---|---|---|
| a | 0,30 | 12 : 1 | 130 | 160 |
| b | 0,40 | 9 : 1 | 130 | 160 |
| c | 0,50 | 7 : 1 | 130 | 160 |
| d | 0,60 | 6 : 1 | 130 | 160 |

| | iso-Butyr-<br>aldehyd<br>% | iso-<br>Butanol<br>% | Di-iso-<br>Butylether<br>% | iso-Butyl-<br>iso-butyrat<br>% |
|---|---|---|---|---|
| a | 0,1 | 99,7 | ≤ 0,05 | < 0,02 |
| b | 0,1 | 99,7 | ≤ 0,05 | < 0,02 |
| c | 0,1 | 99,7 | ≤ 0,05 | 0,05 |
| d | 0,2 | 99,5 | 0,1 | 0,1 |

Die Versuche 3 und 4 wurden als kurzfristige Unterbrechungen in den Versuch 2 zwischengeschaltet.

Man kann den Tabellen 4 bis 7 entnehmen, daß die Hydrierung von gesättigten und ungesättigten Aldehyden im gleichen Reaktor über den gleichen erfindungsgemäßen Katalysatoren mit sehr gutem Resultat möglich ist.

**Patentansprüche**

1. Verfahren zur Herstellung von gesättigten Alkoholen durch Hydrierung von Aldehyden in der Gasphase in mehreren Stufen, wobei man in der ersten Stufe einen körnigen, alkalisch eingestellten Kupferkatalysator und in der zweiten Stufe einen körnigen, Nickel enthaltenden Katalysator verwendet,
dadurch gekennzeichnet,
daß
   - über 85 % der Hydrierreaktionen in der ersten Stufe durchgeführt werden und
   - das Trägermaterial des Katalysators der zweiten Stufe Korngrößen von 1 bis 10 mm aufweist und an der Oberfläche mindestens 0,05 mmol/g saure Zentren enthält, wobei die sauren Zentren eine Säurestärke $H_o$ von 2,8 bis 4,8 besitzen.

2. Verfahren nach Anspruch 1,
dadurch gekennzeichnet,
daß 90 bis 98 % der Hydrierreaktionen in der ersten Stufe durchgeführt werden.

12

3. Verfahren nach Anspruch 1,
   dadurch gekennzeichnet,
   daß das Trägermaterial des Katalysators der zweiten Stufe 0,1 bis 0,4 mmol/g saure Zentren aufweist.

4. Verfahren nach Anspruch 1
   dadurch gekennzeichnet,
   daß das Trägermaterial des Katalysators der zweiten Stufe aus Oxiden des Siliciums und/oder Aluminiums besteht und eine spezifische innere Oberfläche von mindestens 150 $m^2$/g aufweist.

5. Verfahren nach Anspruch 1,
   dadurch gekennzeichnet,
   daß die Hydrierung in der zweiten Stufe an einem Nickelkatalysator durchgeführt wird.

6. Verfahren nach Anspruch 1,
   dadurch gekennzeichnet,
   daß die Hydrierung in der zweiten Stufe an einem Nickel-Kupfer-Katalysator durchgeführt wird.

7. Verfahren nach Ansprüchen 5 und 6,
   dadurch gekennzeichnet,
   daß randzonenimprägnierte Katalysatoren eingesetzt werden.

8. Verfahren nach Anspruch 1,
   dadurch gekennzeichnet,
   daß das Volumen des Katalysators der zweiten Stufe 20 bis 70 % des gesamten Katalysatorvolumens ausmacht.

9. Verfahren nach Anspruch 8,
   dadurch gekennzeichnet,
   daß das Volumen 25 bis 45 % des gesamten Katalysatorvolumens beträgt.

10. Verfahren nach Anspruch 1,
    dadurch gekennzeichnet,
    daß die Hydrierung in einer dritten Stufe an einem körnigen Palladiumkatalysator durchgeführt wird.

11. Verfahren nach Anspruch 10,
    dadurch gekennzeichnet,
    daß das Volumen des Palladiumkatalysators 5 bis 20 % des gesamten Katalysatorvolumens ausmacht.

12. Verfahren nach Anspruch 10,
    dadurch gekennzeichnet,
    daß ein randzonenimprägnierter Palladiumkatalysator eingesetzt wird.

13. Verfahren nach Anspruch 1,
    dadurch gekennzeichnet,
    daß die Eingangstemperatur der ersten Stufe 125 bis 160 °C beträgt und Wasserstoff und Aldehyd im Volumenverhältnis von 100 : 1 bis 3 : 1 eingesetzt werden.

**Claims**

1. Process for preparing saturated alcohols by hydrogenation of aldehydes in the gas phase in a plurality of stages, with a granular copper catalyst which has been made alkaline being used in the first stage and a granular nickel-containing catalyst being used in the second stage, characterized in that
   - more than 85 % of the hydrogenation reactions are carried out in the first stage and
   - the carrier material of the catalyst of the second stage has particle sizes of from 1 to 10 mm and on the surface contains at least 0.05 mmol/g of acid centres, with the acid centres having an acid strength $H_o$ of from 2.8 to 4.8.

EP 0 470 344 B1

2. Process according to Claim 1, characterized in that from 90 to 98 % of the hydrogenation reactions are carried out in the first stage.

3. Process according to Claim 1, characterized in that the carrier material of the catalyst of the second stage has from 0.1 to 0.4 mmol/g of acid centres.

4. Process according to Claim 1, characterized in that the carrier material of the catalyst of the second stage comprises oxides of silicon and/or aluminium and has a specific internal surface area of at least 150 $m^2$/g.

5. Process according to Claim 1, characterized in that the hydrogenation in the second stage is carried out over a nickel catalyst.

6. Process according to Claim 1, characterized in that the hydrogenation in the second stage is carried out over a nickel-copper catalyst.

7. Process according to Claim 5 or 6, characterized in that surface-zone-impregnated catalysts are used.

8. Process according to Claim 1, characterized in that the volume of the catalyst of the second stage makes up from 20 to 70 % of the total catalyst volume.

9. Process according to Claim 8, characterized in that the volume is from 25 to 45 % of the total catalyst volume.

10. Process according to Claim 1, characterized in that the hydrogenation in the third stage is carried out over a granular palladium catalyst.

11. Process according to Claim 10, characterized in that the volume of the palladium catalyst makes up from 5 to 20 % of the total catalyst volume.

12. Process according to Claim 10, characterized in that a surface-zone-impregnated palladium catalyst is used.

13. Process according to Claim 1, characterized in that the inlet temperature of the first stage is from 125 to 160 °C and hydrogen and aldehyde are used in a volume ratio of 100 : 1 to 3 : 1.

**Revendications**

1. Procédé de préparation d'alcools saturés par hydrogénation d'aldéhydes en phase gazeuse et en plusieurs stades, en utilisant dans le premier stade un catalyseur granuleux au cuivre, ajusté à alcalinité et, dans le second stade un catalyseur granuleux renfermant du nickel,
   caractérisé en ce que
   - l'on effectue dans le premier stade plus de 85 % des réactions d'hydrogénation et
   - que la matière de support du catalyseur du second stade présente des grosseurs de grain de 1 à 10 mm et renferme sur la surface au moins 0,05 mmole par gramme de centres acides possédant une force d'acidité $H_o$ de 2,8 à 4,8.

2. Procédé selon la revendication 1,
   caractérisé en ce que l'on effectue dans le premier stade de 90 à 98 % des réactions d'hydrogénation.

3. Procédé selon la revendication 1,
   caractérisé en ce que la matière de support du catalyseur du second stade présente de 0,1 à 0,4 mmole par gramme de centres acides.

4. Procédé selon la revendication 1,
   caractérisé en ce que la matière de support du catalyseur du second stade est constituée par des oxydes du silicium et/ou de l'aluminium et qu'elle présente une surface interne spécifique d'au moins 150 $m^2$ par gramme.

14

5. Procédé selon la revendication 1,
   caractérisé en ce que l'hydrogénation dans le second stade est effectuée sur un catalyseur au nickel.

6. Procédé selon la revendication 1,
   caractérisé en ce que l'hydrogénation dans le second stade est effectuée sur un catalyseur au nickel et au cuivre.

7. Procédé selon les revendications 5 et 6,
   caractérisé en ce que l'on utilise des catalyseurs imprégnés dans les zones marginales.

8. Procédé selon la revendication 1,
   caractérisé en ce que le volume du catalyseur du second stade est de 20 à 70 % du volume global de catalyseur.

9. Procédé selon la revendication 8,
   caractérisé en ce que le volume est de 25 à 45 % du volume global de catalyseur.

10. Procédé selon la revendication 1,
    caractérisé en ce que l'hydrogénation est effectuée dans un troisième stade sur un catalyseur granuleux au palladium.

11. Procédé selon la revendication 10,
    caractérisé en ce que le volume du catalyseur au palladium est de 5 à 20 % du volume global de catalyseur.

12. Procédé selon la revendication 10,
    caractérisé en ce que l'on utilise un catalyseur au palladium imprégné sur les zones marginales.

13. Procédé selon la revendication 1,
    caractérisé en ce que la température d'entrée du premier stade est de 125 à 160°C et que l'on utilise l'hydrogène et l'aldéhyde dans une proportion volumétrique de 100 : 1 à 3 : 1.